(19) Europäisches Patentamt
European Patent Office
Office européen des brevets

(11) Numéro de publication: **0 089 297**
**B1**

(12) **FASCICULE DE BREVET EUROPEEN**

(45) Date de publication du fascicule du brevet: **12.06.85**

(51) Int. Cl.⁴: **C 07 D 251/34**, C 08 G 18/02

(21) Numéro de dépôt: **83420032.1**

(22) Date de dépôt: **02.03.83**

(54) **Procédé de préparation de polyisocyanates polyisocyanurates par cyclotrimérisation catalytique de polyisocyanates.**

(30) Priorité: **04.03.82 FR 8203799**

(43) Date de publication de la demande: **21.09.83 Bulletin 83/38**

(45) Mention de la délivrance du brevet: **12.06.85 Bulletin 85/24**

(84) Etats contractants désignés: **AT BE CH DE FR GB IT LI LU NL SE**

(56) Documents cités:
**FR-A-1 304 301**
**FR-A-2 235 147**

(73) Titulaire: **RHONE-POULENC SPECIALITES CHIMIQUES, "Les Miroirs" 18, Avenue d'Alsace, F-92400 Courbevoie (FR)**

(72) Inventeur: **Robin, Jean, 21, rue Duguesclin, F-69006 Lyon (FR)**

(74) Mandataire: **Chichery, Guy et al, RHONE-POULENC RECHERCHES Service Brevets Chimie et Polymères Centre de Recherches de Saint-Fons B.P. 62, F-69192 St-Fons Cédex (FR)**

**EP 0 089 297 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne un procédé de préparation de polyisocyanates polyisocyanurates, par cyclotrimérisation catalytique partielle de polyisocyanates, cette réaction étant ultérieurement et volontairement arrêtée lorsque la teneur en trimère atteint la valeur désirée. Plus précisément, la présente invention concerne essentiellement la manière d'opérer la désactivation du catalyseur, lors de l'emploi d'un catalyseur constitué par un composé à groupement aminosilylé.

On connaît de nombreux catalyseurs qui permettent de cyclotrimériser en polyisocyanates polyisocyanurates les polyisocyanates aliphatiques ou aromatiques. Chaque catalyseur utilisé est, par la suite, désactivé selon une technique appropriée propre à l'agent catalytique utilisé.

On sait, par exemple, cyclotrimériser partiellement les polyisocyanates simples, aliphatiques ou aromatiques, ou les polyisocyanates adducts, à l'aide de catalyseurs basiques tels que les amines tertiaires (brevet allemand N° 951168), les dérivés des métaux alcalins ou alcalino-terreux tels qu'hydroxyde, carbonate, alcoolate, etc. (brevet français N° 1190065), les hydroxydes d'ammonium quaternaire (brevets français N$^{os}$ 1204697 et 1566256; demandes de brevets européens N$^{os}$ 3765 et 10589), les catalyseurs à groupement éthylène-imine (brevets français N$^{os}$ 1401513 et 2230642) et enfin les bases de Mannich en général obtenues à partir de phénol, d'aldéhyde et d'amine secondaire (brevets français N$^{os}$ 2290459 et 2332274). Ces catalyseurs basiques sont, en général, tout naturellement désactivés par introduction d'un composé acide tel qu'un acide (acide phosphorique, acide chlorhydrique, etc.), un chlorure d'acide (chlorure d'acétyle, chlorure de benzoyle, chlorure p-toluènesulfonique, etc.). On peut également détruire l'activité catalytique des bases de Mannich soit par un traitement thermique, soit par addition d'un agent alkylant (sulfate de diméthyle, iodure de métyle, etc.).

Par ailleurs, on sait également préparer des polyisocyanates polyisocyanurates par cyclotrimérisation catalytique, en utilisant des phosphines à titre de catalyseur (brevets français N$^{os}$ 1510342 et 2023423, demande de brevet allemand N° 1934763). La désactivation des phosphines après la réaction de cyclotrimérisation est, en général, effectuée par addition d'agent alkylant ou acylant, ou encore par addition de soufre.

La manière d'arrêter la réaction de cyclotrimérisation catalytique a, dans la réalité industrielle, une importance très grande. Le désactivateur, en détruisant le catalyseur, va se retrouver, après transformation, ainsi d'ailleurs que le catalyseur détruit, dans le polyisocyanate polyisocyanurate, à l'état d'espèces plus ou moins complexes, mais qui auront inéluctablement une influence parfois non négligeable sur la coloration du produit, sa stabilité, etc.

En outre, les agents acides sont corrosifs vis-à-vis de l'appareillage utilisé à l'échelle industrielle, tant pour la fabrication que pour le stockage.

Par ailleurs, la titulaire a mis au point un nouveau système catalytique permettant de cyclotrimériser, en polyisocyanates polyisocyanurates, les polyisocyanates aliphatiques ou cycloaliphatiques, ces polyisocyanates étant simples ou adducts. C'est ainsi que l'utilisation de composés à groupements aminosilylés à titre de catalyseurs, tels que les monoaminosilanes, les diaminosilanes, les silylurées et les silazanes permettent de réaliser la réaction de cyclotrimérisation sans période d'induction, et de manière régulière à une température relativement modérée, ce qui réduit dans une proportion très importante la formation de polyisocyanate dimère. Ces catalyseurs, qui seront précisés dans la suite de l'exposé, sont décrits dans EP-A N° 57653. Dans cette publication, il a été indiqué que la désactivation du catalyseur était assurée par addition d'un composé acide, tel qu'un acide fort ou un halogénure d'acide. On s'est cependant aperçu que l'emploi de ces désactivateurs entraînait quelques inconvénients (coloration, corrosion, effet catalytique éventuel des produits de corrosion, etc.).

Il a maintenant été trouvé, et c'est ce qui constitue l'objet de la présente invention, un procédé de préparation de polyisocyanates polyisocyanurates par cyclotrimérisation catalytique de polyisocyanates, en utilisant à titre de catalyseurs des composés à groupements aminosilylés, caractérisé en ce que, lorsque la teneur désirée en groupement isocyanurate est atteinte, le catalyseur est détruit par addition d'un composé désactivateur, choisi parmi: a) les composés organiques A, porteurs au moins d'un groupement hydroxyle, ou b) les composés résultant de la réaction de groupement isocyanate sur le composé organique A, le composé organique A, éventuellement porteur de groupement ou atomes inertes vis-à-vis des groupements isocyanates, étant choisi parmi les énols, les alcools, les phénols, les oximes, ou c) les composés porteurs de groupement(s) hydroxysilylé(s).

L'utilisation de ces désactivateurs procure l'avantage suivant: inhibition de la coloration et de la corrosion. On peut également, selon la volatilité du désactivateur introduit, éliminer totalement ce dernier, après avoir stoppé la réaction de cyclotrimérisation.

L'invention permet donc ainsi de préparer de nouveaux polyisocyanates polyisocyanurates qui constituent également un objet de la présente invention.

Par ailleurs, la possibilité de désactiver le catalyseur par l'utilisation de composés à groupements hydroxyles est totalement inattendue. On sait, en effet, que les catalyseurs de cyclotrimérisation catalytique tels que les amines tertiaires, les dérivés des métaux alcalins ou alcalino-terreux, les bases de Mannich, voient leurs activités catalytiques accrues par l'adjonction d'un ester carbamique jouant le rôle de cocatalyseur et directement habituellement généré *in situ*, dans le milieu réactionnel par addition d'alcool (en général secondaire, mais pouvant être primaire) ou de phénol (cf. brevets français N$^{os}$ 1172576, 1304301, 1566256 et 2290459). Un tel rôle est par ailleurs également

bien mis en évidence par I.C. Kogon [«Journal of American Chemical Society», *78*, pp. 4911 à 4913 (1956)] et par J.E. Kresta et H.K. Hsieh [«Makromol. Chem.», *1978*, 179 (11) 2779-82].

L'agent désactivateur peut être un composé organique A porteur d'un groupement hydroxyle et choisi parmi les énols, les alcools primaires, secondaires ou tertiaires, les polyols primaires, secondaires ou tertiaires, les phénols, les polyphénols, les oximes, les composés à groupements hydroxysilylés tels que les silanols, les silanediols, les siloxanes ou les polysiloxanes à groupement(s) hydroxysilylé(s). Bien entendu, comme on l'a déjà dit, outre le groupement hydroxyle, le composé A peut éventuellement contenir tout groupement ou atome inerte, vis-à-vis des groupements isocyanates, tels que des groupements ester, éther, amide, des groupements organométalliques ou organométalloïdiques, etc.

On mettra en œuvre, à titre d'énols, des composés ayant au plus 10 atomes de carbone tels que les β-dicétones, les β-cétoesters et les β-cyanoesters. A titre illustratif, on pourra citer l'acétylacétone, l'acétylacétate d'éthyle ou de méthyle ou de pentyle, le cyanoacétate d'éthyle.

On mettra en œuvre, à titre de monoalcools, des carbinols, primaires, secondaires ou tertiaires ayant, en général, de 1 à 8 atomes de carbone. Ces alcools peuvent éventuellement contenir des substituants inertes vis-à-vis des groupements isocyanates tels que des groupements éther, ester, amide, etc. Ces alcools peuvent ainsi être des hydroxyorganosilanes ou des hydroxyalkylsilanes. On reviendra dans la suite de l'exposé sur de tels composés.

Avantageusement, si on désire éliminer totalement le reliquat du désactivateur, on utilisera dans le cadre du procédé de la présente invention des monoalcools simples purement hydrocarbonés et peu condensés en carbone (au plus 6 atomes de carbone), primaires ou secondaires, tels que le méthanol, l'éthanol, le propanol, le n-butanol, l'isopropanol, le butanol secondaire, etc. Préférentiellement, on utilisera des monoalcools primaires ou secondaires, ayant de 3 à 6 atomes de carbone, et dont la volatilité n'est de ce fait pas trop importante, tels que le butanol ou l'isopropanol.

On peut également utiliser, à titre de composé organique A, un polyol éventuellement substitué par un ou plusieurs groupements inertes tels que précédemment définis. Dans un tel contexte, on peut citer:

- le glycérol,
- le propylèneglycol-1,3,
- le butanediol-1,4,
- le triéthylèneglycol,
- l'octanediol-1,3,
- le butynediol-1,4,
- le triméthylolpropane,
- l'éther monoéthylique ou méthylique du diéthylèneglycol (diglyme).

Les polyols ont, en général, de 2 à 12 atomes de carbone et préférentiellement de 2 à 8 atomes de carbone. On notera, dans ce contexte, que l'utilisation d'un désactivateur lourd et donc non volatil, tel qu'un polyol ou un alcool lourd, permet de récupérer les restes du catalyseur silicié dans les culots de distillation, après élimination de l'excès de diisocyanate et du polyisocyanate polyisocyanurate. On peut ainsi, après les traitements appropriés, recycler tout le silicium engagé avec le catalyseur.

Les phénols utilisables peuvent être des phénols mono- ou polycycliques renfermant éventuellement un ou plusieurs groupements phénoliques, et pouvant comporter divers substituants inertes vis-à-vis des groupements isocyanates, tels que des groupements alkyle, ester, éther, des atomes d'halogène, etc. A titre illustratif, on pourra citer parmi les phénols utilisables:

- le phénol,
- les crésols,
- les xylénols,
- le nonylphénol,
- les tertiobutylphénols,
- les dihydroxybenzènes,
- le dihydroxy-4,4' biphényle,
- le dihydroxy-4,4' diphénylméthane,
- l'hydroxynaphtalène,
- le naphtalènediol, etc.

Les oximes utilisables peuvent être des cétoximes ou des aldoximes obtenues par réaction d'hydroxylamine avec des aldéhydes ou des cétones linéaires ou cyclaniques ayant au plus 10 atomes de carbone; parmi les oximes on peut citer: l'acétone-oxime, la méthyléthylcétone-oxime, la cyclohexanone-oxime, l'hexanone-2 oxime, la cinnamaldéhyde-oxime, etc.

Parmi les composés à groupements hydroxysilylés, on pourra citer, à titre illustratif:

- le triméthylsilanol,
- le diméthylsilanediol,
- le triéthylsilanol,
- le diéthylsilanediol,
- le triphénylsilanol,
- le diphénylsilanediol,
- le dihydroxydiméthyldisiloxane,
- le dihydroxydiphényldisiloxane,
- le bis-α, ω-dihydroxy(octaphényltétrasiloxane), etc.

On peut évidemment utiliser, dans le cadre de la présente invention, des composés organosiliciques porteurs de groupements hydroxyles non directement reliés à un atome de silicium. On pourra ainsi utiliser des hydroxyorganosilanes ou des hydroxyorganopolysiloxanes tels que:

- le triméthyl(hydroxyméthyl)silane,
- l'(hydroxybutyl)triméthylsilane,
- le bis(hydroxypropyl)diméthylsilane,
- l'hydroxyphényltriméthylsilane, etc.

Les composés organosiliciques à groupements hydroxyles sont par exemple décrits dans l'ouvrage de Walter Noll: «Chemistry and technology of silicones», édition anglaise 1968.

On peut également utiliser, à titre d'agent désactivateur, un composé résultant de la réaction d'un isocyanate sur un composé organique A tel que précédemment défini. On observera tout de suite qu'un tel composé est, en général, obtenu directement, *in situ*, dans le milieu réactionnel encore très

riche en groupements isocyanate, et cela lors de l'addition du composé A. On pourra donc ainsi, selon une variante du procédé de l'invention, introduire directement dans le milieu réactionnel, et à titre d'agent désactivateur, un uréthanne d'alkyle ou d'aryle, ou encore un o-silyluréthanne, le groupement silylé étant directement lié à l'atome d'oxygène. Selon une variante préférée, on introduit à titre de désactivateur un uréthanne issu d'un composé porteur d'au moins un groupement isocyanate et d'un monoalcool primaire ou secondaire ayant de 1 à 8 atomes de carbone.

A titre avantageux, on préfère utiliser dans le cadre de la présente invention un monoalcool primaire, secondaire ayant de 3 à 6 atomes de carbone. L'emploi de butanol ou d'isopropanol se révèle particulièrement approprié.

La quantité d'agent désactivateur mise en œuvre peut être variable. Celle-ci n'est pas critique mais, bien entendu, elle dépendra de la quantité de catalyseur initialement introduit dans le polyisocyanate.

En général, la quantité d'agent désactivateur est telle que le rapport molaire entre l'agent désactivateur et le catalyseur soit compris entre 0,5 et 2 et préférentiellement entre 0,8 et 1,5. Avantageusement, on utilise un rapport molaire voisin de 1.

Le composé initiant la réaction catalytique peut être un composé à groupement aminosilylé et ayant pour formule:

$$R_{(4-n)}\text{———}Si\text{-}[NR'R'']_n \qquad (I)$$

dans laquelle les divers symboles représentent respectivement:

– R: un radical monovalent de nature hydrocarbonée, aliphatique, cycloaliphatique, saturé ou insaturé, aryle, aralkyle ou alkylaryle, éventuellement substitué par des atomes d'halogène ou des groupements CN, deux radicaux R pouvant constituer ensemble un radical hydrocarboné divalent,

– R': un radical monovalent choisi parmi les radicaux R, $SiR_3$, ou les radicaux amide de formule:

$$-CO-\underset{\displaystyle R}{\underset{|}{N}}-R'''$$

R''' représentant R ou $SiR_3$, R ayant la signification préalablement donnée, le radical R' pouvant éventuellement, lorsqu'il ne représente pas un groupement amide ou un groupement $SiR_3$, constituer avec le radical R'' un radical hydrocarboné divalent,

– R'': un radical monovalent ayant la même signification que le radical R ou un atome d'hydrogène lorsque R' n'est pas un radical amide,

– n: un nombre entier égal à 1 ou 2; lorsque n est égal à 2, R' est un radical R.

Le catalyseur, qui peut être un aminosilane, un diaminosilane, une silylurée ou un silazane, est plus précisément représenté par la formule (I), dans laquelle les divers symboles représentent respectivement:

– R: un radical alkyle, alkényle ou halogénoalkyle ou halogénoalkényle ayant de 1 à 5 atomes de carbone et comportant de 1 à 6 atomes de chlore et/ou de fluor, des radicaux cycloalkyles, cycloalkényles et halogénocycloalkyles, halogénocycloalkényles ayant de 3 à 8 atomes de carbone et contenant de 1 à 4 atomes de chlore et/ou de fluor, des radicaux aryles, alkylaryles et halogénoaryles ayant de 6 à 8 atomes de carbone et contenant de 1 à 4 atomes de chlore et/ou de fluor, des radicaux cyanoalkyles ayant de 3 à 4 atomes de carbone;

– deux symboles R portés par un même atome de silicium constituent entre eux un radical divalent ayant de 1 à 4 atomes de carbone.

– R': un radical monovalent choisi parmi les radicaux R, $SiR_3$, et CO(NR)–R''', R''' représentant R ou $SiR_3$, R ayant la signification plus précise qui vient juste d'être donnée ci-dessus; R' pouvant constituer avec R'' un radical alkylène ayant de 4 à 6 atomes de carbone;

– R'': un radical alkyle ou alkényle ayant de 1 à 4 atomes de carbone, un radical cycloalkyle ou cycloalkényle ayant de 4 à 6 atomes de carbone nucléaire, un radical phényle ou tolyle ou xylyle, ou un atome d'hydrogène lorsque R' n'est pas un groupement amide.

Les composés aminosilylés, de formule (I), utilisés préférentiellement à titre de catalyseurs de cyclotrimérisation sont ceux dans la formule desquels les divers symboles représentent respectivement:

– R: un radical méthyle, éthyle, propyle, vinyle, phényle, ces radicaux pouvant éventuellement être chlorés et/ou fluorés,

– R': un radical alkyle choisi parmi les radicaux méthyle, éthyle, propyle ou butyle, un radical $SiR_3$, R ayant l'une des significations qui viennent juste d'être définies, un radical carbonamide choisi parmi: CO–NR–R, –CO–NR–$SiR_3$, R ayant l'une des significations qui viennent juste d'être données,

– R'': un radical méthyle, éthyle, propyle ou butyle, ou un atome d'hydrogène.

Enfin R' et R'' peuvent ensemble constituer un radical butylène ou pentylène.

Comme on l'a déjà dit, le catalyseur de cyclotrimérisation peut être un aminosilane, un diaminosilane, une monosilylurée, une disilylurée ou un silazane. Il est facile de déterminer la nature chimique exacte des divers composés à groupements aminosilylés utilisables, étant donné les diverses significations données précédemment aux divers radicaux R, R', R'', R'''. On notera en particulier que l'utilisation de silylurée obtenue par réaction d'amine secondaire et d'isocyanates-N silylés n'est pas envisagée. Ces silylurées sont impropres dans le procédé de cyclotrimérisation catalytique, puisque libérant l'isocyanate silylé lors du chauffage.

Le composé à groupement aminosilylé sera un aminosilane lorsque n sera égal à 1 et lorsque R' représentera un radical R, les radicaux R et R'' ayant l'une des significations préalablement données, deux radicaux R pouvant constituer ensemble un radical divalent ou bien encore R' et R'' pouvant constituer ensemble un radical divalent. Parmi les aminosilanes on citera:

– le méthylaminotriméthylsilane,

– le diméthylaminotriméthylsilane,
– le diéthylaminotriméthylsilane,
– le dibutylaminotriméthylsilane,
– le diéthylaminodiméthylvinylsilane,
– le diéthylaminodiméthylphénylsilane.

Le composé à groupement aminosilylé sera un diaminosilane lorsque n sera égal à 2 et lorsque R' représentera le radical R, les radicaux R et R" ayant l'une des significations préalablement données, deux radicaux R pouvant constituer ensemble un radical divalent ou bien encore R' et R" pouvant constituer un radical divalent. Parmi les diaminosilanes, on citera:

– le bisdiméthylaminodiméthylsilane,
– le bisdiméthylaminodiméthylsilane,
– le bisdibutylaminodiméthylsilane,
– le bisdiméthylaminométhylphénylsilane.

Le composé à groupement aminosilylé sera une silylurée lorsque n sera égal à 1 et lorsque R' représentera le groupement carbonamide:

$$-\underset{\underset{\displaystyle O}{|}}{C}-NRR'''$$

où R''' représente un radical R ou $SiR_3$, les radicaux R et R" ayant l'une des significations préalablement données, 2 radicaux R pouvant constituer ensemble un radical divalent ou les deux radicaux R' et R" (R' représentant alors R) pouvant constituer ensemble un radical divalent. Parmi les silylurées, on citera:

– la N,méthyl-N,triméthylsilyl-N',méthyl-N',butylurée,
– la N,triméthylsilyl-N,méthyl-N',N',diméthylurée,
– la N,triméthylsilyl-N,éthyl-N',N',diméthylurée,
– la N,triméthylsilyl-N,butyl-N',butyl-N',triméthylsilylurée.

Le composé à groupement aminosilylé sera un silazane lorsque n sera égal à 1 et lorsque R' représentera un groupement $SiR_3$.

Les silazanes peuvent être symétriques ou dissymétriques; on emploie préférentiellement les disilazanes symétriques, les deux groupements $SiR_3$ étant identiques.

Parmi les disilazanes utilisables, on citera:

– l'hexaméthyldisilazane,
– l'heptaméthyldisilazane,
– le diéthyl-1,3 tétraméthyl-1,1,3,3 disilazane,
– le divinyl-1,3 tétraméthyl-1,1,3,3 disilazane,
– l'hexaméthyldisilazane,
– le diphényl-1,3 tétraméthyl-1,1,3,3 disilazane, etc.

Enfin, on mentionnera tout particulièrement parmi les disilazanes l'hexaméthyldisilazane, l'heptaméthyldisilazane qui se révèlent être des catalyseurs tout particulièrement avantageux.

Dans le procédé de la présente invention, on peut cyclotrimériser en polyisocyanate polyisocyanurate tout polyisocyanate simple ou adduct, de nature aliphatique, cycloaliphatique ou aromatique, et cela à condition de bien choisir, en tant que catalyseur, le composé à groupement aminosilylé apte à cette réaction.

C'est ainsi que la cyclotrimérisation catalytique de polyisocyanates simples ou adducts, et dont les groupements isocyanates ne sont pas directement reliés à un noyau aromatique, peut être aisément effectuée en mettant en œuvre, en tant que catalyseur, un aminosilane, un diaminosilane, une silylurée ou un silazane, tels que précédemment définis.

Dans ce contexte, parmi les diisocyanates aliphatiques ou cycloaliphatiques, on citera:

– le tétraméthylènediisocyanate,
– le pentaméthylènediisocyanate,
– l'hexaméthylènediisocyanate,
– le diisocyanato-1,2 cyclohexane,
– le diisocyanato-1,4 cyclohexane,
– le bis(isocyanateméthyl)-1,2 cyclobutane,
– le bis(isocyanato-4 cyclohexyl)méthane,
– le triméthyl-3,3,5 isocyanatométhyle-5 isocyanato-1 cyclohexane.

Parmi ceux-ci, on mentionnera tout particulièrement l'hexaméthylènediisocyanate.

Enfin, on peut citer, parmi les polyisocyanates adducts ou prépolymères utilisables à titre de polyisocyanates de nature aliphatique, les polyisocyanates modifiés qui sont obtenus en faisant réagir un excès de polyisocyanate aliphatique ou cycloaliphatique sur un composé comportant au moins deux groupements réactifs vis-à-vis des groupements isocyanates, tels qu'une diamine, un diacide, etc. Les polyisocyanates modifiés qui peuvent être mélangés avec des polyisocyanates simples peuvent comporter des groupements urée, biuret, ester, siloxane, etc.

On peut également, dans le cadre du procédé de la présente invention, cyclotrimériser en polyisocyanate polyisocyanurate tout polyisocyanate simple ou adduct de nature aromatique, c'est-à-dire ceux dans lesquels le groupement NCO est directement lié à un groupement aromatique. Pour y parvenir, on utilisera, en tant que catalyseurs à groupements aminosilylés, les aminosilanes, les diaminosilanes ou les silylurées telles que précédemment définies.

Parmi les diisocyanates aromatiques utilisables, on citera:

– le diisocyanato-1,4 benzène,
– les diisocyanatotoluènes(–2,4 et –2,6) ou leurs mélanges,
– le diisocyanato-4,4' diphénylméthane,
– le diisocyanato-4,4' diphényléther,
– les polyméthylène-, polyphénylènepolyisocyanates, etc.

On peut également utiliser à titre de polyisocyanate aromatique tout polyisocyanate adduct résultant de la polycondensation d'un excès de polyisocyanate sur un composé polyfonctionnel tel qu'une diamine, un diacide, etc. Les polyisocyanates modifiés qui peuvent être mélangés avec des polyisocyanates simples peuvent comporter des groupements urée, biuret, ester, siloxane, etc.

La quantité d'agent catalytique introduite dans l'isocyanate peut être variable; exprimée pondéralement par rapport à l'isocyanate engagé, elle est habituellement comprise entre 0,1 et 10% et de préférence entre 0,5 et 5%; on peut éventuellement

introduire de petites quantités complémentaires de catalyseur durant la réaction.

Le procédé de cyclotrimérisation en polyisocyanate polyisocyanurate peut être effectué par simple chauffage des réactifs, à une température en général comprise entre 50 et 180°C, de préférence entre 80 et 130°C, et habituellement autour de 100°C.

Il est également possible, le cas échéant, d'effectuer la réaction de cyclotrimérisation en milieu solvant, ce dernier pouvant être un solvant peu polaire, par exemple un hydrocarbure aliphatique ou aromatique, ou un ester ou un éther. On peut alors introduire le catalyseur dans le solvant et introduire cette solution dans l'isocyanate. On peut également introduire évidemment la solution catalytique dans l'isocyanate. Avantageusement, le procédé est effectué sans solvant.

Lorsque la teneur en isocyanurate atteint la valeur désirée, on ajoute le désactivateur dont la nature et la proportion ont été précédemment définies. Cete introduction est en général effectuée à une température comprise entre 50 et 180°C et préférentiellement entre 80 et 130°C. Selon un mode opératoire commode, l'ajout du désactivateur est réalisé à la même température que celle à laquelle a été faite la réaction de cyclotrimérisation. La désactivation du catalyseur s'effectue très rapidement en quelques minutes.

On peut alors éventuellement éliminer le polyisocyanate monomère excédentaire par tout moyen connu et parvenir à un polyisocyanate polyisocyanurate renfermant une teneur excessivement réduite en isocyanate monomère, ainsi qu'une faible teneur en isocyanate dimère.

Les polyisocyanates polyisocyanurates tels que ceux issus d'hexaméthylènediisocyanate sont des composés bien connus et particulièrement intéressants comme constituants de base pour vernis et peinture.

Les exemples qui suivent illustrent l'invention.

*Exemple 1:*

Dans un ballon on charge 400 g (2,38 mol) de diisocyanato-1,6 hexane.

On chauffe au bain d'huile à 95°C et on ajoute 8 g (0,05 mol) d'hexaméthyldisilazane et on maintient à 100°C pendant 2 h. A ce moment-là, on dose 1,003 groupement isocyanate pour 100 g, soit une disparition de 15% environ des groupes NCO présents initialement. On fractionne la masse réactionnelle en parties aliquotes de 40 g, auxquelles on ajoute, pour arrêter la réaction, une petite quantité soit d'un composé porteur de groupement hydroxyle, soit d'un composé obtenu par réaction de groupement isocyanate sur un composé porteur de groupement hydroxyle.

La quantité d'alcool ou d'adduct (uréthanne, etc.) ajoutée correspond à un groupe OH (ou uréthanne) par mole d'hexaméthyldisilazane.

L'arrêt de la réaction est effectif en quelques minutes.

Ces fractions sont ensuite maintenues à 100°C pendant 20 h pour en vérifier la stabilité.

Les bloqueurs utilisés sont:

– le n-butanol,
– l'isopropanol,
– le phénol,
– le glycérol,
– le triméthylsilanol,
– le di-n-butyluréthanne de l'hexaméthylène-diisocyanate,
– le diisopropyluréthanne de l'hexaméthylène-diisocyanate.

Dans tous les cas, on constate que la teneur en NCO est restée pratiquement constante, alors que celle d'un témoin maintenu 20 h à 100°C, sans addition de bloqueur, voit sa viscosité augmenter fortement, son titre en NCO étant de 0,538 groupement NCO/100 g au bout de ce laps de temps à 100°C.

*Exemple 2:*

Dans un ballon on charge 336 g (2 mol) de diisocyanatohexane.

On chauffe au bain d'huile à 90-95°C et on ajoute 4,7 g (0,04 mol) de triméthylsilyldiméthylamine; on maintient à 100°C pendant 2 h; à ce moment-là, on dose 1,030 groupement isocyanate pour 100 g.

La masse réactionnelle est alors fractionnée en parties aliquotes de 51 g, auxquelles on ajoute, pour arrêter la réaction, une petite quantité d'un des composés suivants:

– hydroquinone,
– hydroquinone B,
– cyclohexanone-oxime,
– acétylacétate d'éthyle,
– acétylacétone.

La quantité de bloqueur ajoutée correspond à 1 groupe OH par mole d'aminosilane, sauf pour hydroquinone B, où l'on met 2 groupes OH par mole d'aminosilane.

On maintient 17 h à 100°C pour vérifier si la réaction est bien arrêtée; on constate qu'au bout de ce laps de temps la teneur en NCO des divers fractions n'a pratiquement pas varié (variation supérieure à 5%).

*Exemple 3:*

Dans un ballon on charge 336 g (2 mol) de diisocyanatohexane.

On chauffe au bain d'huile à 95°C et on ajoute 8,7 g (0,04 mol) de N-butyl N-triméthylsilyl N'N'-diméthylurée. On maintient à 110°C pendant 1 h 20 min. Au bout de ce temps, on dose 1,050 groupement NCO/100 g.

On fractionne aussitôt la masse réactionnelle en parties aliquotes de 51 g, auxquelles on ajoute, pour arrêter la réaction, une quantité donnée de l'un des produits suivants:

– hexanol 1,
– octanediol 1,8,
– paracrésol,
– triméthylsilylméthanol,
– polysiloxane α-ω-dihydroxylé (PM 1700; m = 50 ctsk).

La quantité est telle que l'on ait un groupe OH par mole de silylurée.

Les diverses fractions sont conservées à 100°C

pendant 20 h, et on constate que la teneur en NCO est restée pratiquement inchangée.

*Exemple 4:*

Dans un ballon on charge 84 g (0,5 mol) de diisocyanatohexane.

On chauffe au bain d'huile à 120° C et on ajoute 0,6 g (0,005 mol) de triméthylsilyldiméthylamine. On amène à 145° C et, au bout de 1½ h, on dose 1,005 groupement NCO/100 g.

Sur une moitié du mélange réactionnel, on ajoute 0,65 g d'éthyl-2 hexanol (soit 2 mol/mole d'aminosilane) et on porte 15 min à 180° C.

On maintient ensuite à 100° C pendant 24 h: la teneur en NCO n'a pratiquement pas varié, alors que l'autre partie du mélange réactionnel, non additionnée d'éthylhexanol, est devenue extrêmement visqueuse.

*Exemple 5:*

Dans un ballon on charge 84 g (0,5 mol) de diisocyanatohexane.

On chauffe au bain d'huile à 100° C et on ajoute 1,6 g (0,01 mol) d'hexaméthyldisilazane. On maintient 2 h à 100°C et on dose alors 1,00 groupement NCO/100 g.

On ajoute alors 0,6 g de butanol 1 (soit 0,8 mol/mole de silazane), divise en 2 le mélange et maintient la première partie à 100° C pendant 20 h: la teneur en NCO reste pratiquement la même.

La deuxième partie est refroidie pendant 1 h à 50° C et est conservée ensuite 20 h à 100° C; il n'y a pas non plus évolution de la teneur en NCO.

**Revendications** pour les Etats contractants BE, CH, DE, FR, GB, IT, LI, LU, NL, SE

1. Procédé de préparation de polyisocyanates polyisocyanurates par cyclotrimérisation catalytique de polyisocyanates en utilisant à titre de catalyseurs des composés à groupements aminosilylés, caractérisé en ce que, lorsque la teneur désirée en groupements isocyanurate est atteinte, le catalyseur est détruit par addition d'un composé désactivateur, choisi parmi: a) les composés organiques A porteurs d'au moins un groupement hydroxyle, ou b) les composés résultant de la réaction de groupement isocyanate sur le composé organique A, le composé organique A étant éventuellement porteur de groupements ou atomes inertes vis-à-vis des groupements isocyanates et étant choisi parmi les énols, les alcools, les phénols, les oximes, ou c) les composés organosiliciques porteurs de groupement(s) hydroxysilylé(s).

2. Procédé selon la revendication 1, dans lequel le composé désactivateur est choisi parmi les composés organiques A ou les composés résultant de la réaction de groupement isocyanate sur le composé A, le composé A étant un énol, un alcool primaire, secondaire ou tertiaire, un polyol primaire, secondaire ou tertiaire, un phénol ou un polyphénol, une cétoxime, un silanol, un silanediol, un siloxane ou un polysiloxane à groupement(s) hydroxysilylé(s).

3. Procédé selon l'une des revendications 1 ou 2, dans lequel le composé désactivateur est choisi parmi les monoalcools primaires ou secondaires ayant de 1 à 8 atomes de carbone, ou parmi les uréthannes issus d'un composé porteur d'au moins un groupement isocyanate et d'un monoalcool primaire ou secondaire ayant de 1 à 8 atomes de carbone.

4. Procédé selon l'une des revendications 1 à 3, dans lequel le composé désactivateur est un monoalcool primaire ou secondaire ayant de 3 à 6 atomes de carbone.

5. Procédé selon l'une des revendications précédentes, dans lequel le composé aminosilylé utilisé à titre de catalyseur de cyclotrimérisation catalytique des polyisocyanates est un composé ayant pour formule:

$$R_{(4-n)}—Si[NR'R'']_n \qquad (I)$$

dans laquelle les divers symboles représentent respectivement:

— R: un radical monovalent de nature hydrocarbonée, aliphatique, cycloaliphatique, saturé ou insaturé, aryle, aralkyle ou alkylaryle, éventuellement substitué par des atomes d'halogène ou des groupements CN, deux radicaux R pouvant constituer ensemble un radical hydrocarboné divalent,

— R': un radical monovalent choisi parmi les radicaux R, $SiR_3$, ou les radicaux amide de formule:

$$-CO-\underset{\substack{|\\R}}{N}-R'''$$

R''' représentant R ou $SiR_3$, R ayant la signification préalablement donnée, le radical R' pouvant éventuellement, lorsqu'il ne représente pas un groupement amide ou un groupement $SiR_3$, constituer avec le radical R'' un radical hydrocarboné divalent,

— R'': un radical monovalent ayant la même signification que le radical R, ou un atome d'hydrogène lorsque R' n'est pas un radical amide,

— n: un nombre entier égal à 1 ou 2; lorsque n est égal à 2, R' est un radical R.

6. Procédé selon la revendication 5, dans lequel le composé à groupement aminosilylé a pour formule (I), dans laquelle les divers symboles représentent respectivement:

— R: un radical alkyle, alkényle ou halogénoalkyle ou halogénoalkényle ayant de 1 à 5 atomes de carbone et comportant de 1 à 6 atomes de chlore et/ou de fluor, des radicaux cycloalkyles, cycloalkényles et halogénocycloalkyles, halogénocycloalkényles ayant de 3 à 8 atomes de carbone et contenant de 1 à 4 atomes de chlore et/ou de fluor; des radicaux aryles, alkylaryles et halogénoaryles ayant de 6 à 8 atomes de carbone et contenant de 1 à 4 atomes de chlore et/ou de fluor; des radicaux cyanoalkyles ayant de 3 à 4 atomes de carbone,

— deux symboles R portés par un même atome de silicium constituent entre eux un radical divalent ayant de 1 à 4 atomes de carbone,

— R': un radical monovalent choisi par les radicaux R, $SiR_3$, et CO(NR)−R''', R''' représentant R ou $SiR_3$, R ayant la signification plus précise qui

vient juste d'être donnée ci-dessus, R' pouvant constituer avec R'' un radical alkylène ayant de 4 à 6 atomes de carbone,

– R'': un radical alkyle ou alkényle ayant de 1 à 4 atomes de carbone, un radical cycloalkyle ou cycloalkényle ayant de 4 à 6 atomes de carbone nucléaires, un radical phényle ou tolyle ou xylyle, ou un atome d'hydrogène, lorsque R' n'est pas un groupement amide.

7. Procédé selon l'une des revendications 5 ou 6, dans lequel le composé à groupement aminosilylé est un aminosilane.

8. Procédé selon l'une des revendications 5 ou 6, dans lequel le composé à groupement aminosilylé est un diaminosilane.

9. Procédé selon l'une des revendications 5 ou 6, dans lequel le composé à groupement aminosilylé est une silylurée.

10. Procédé selon l'une des revendications 5 ou 6, dans lequel le composé à groupement aminosilylé est un silazane.

11. Polyisocyanates polyisocyanurates obtenus selon les procédés tels que définis dans l'une des revendications 1 à 10.

**Revendications** pour l'Etat contractant: AT

1. Procédé de préparation de polyisocyanates polyisocyanurates par cyclotrimérisation catalytique de polyisocyanates en utilisant à titre de catalyseurs des composés à groupements aminosilylés, caractérisé en ce que, lorsque la teneur désirée en groupements isocyanurate est atteinte, le catalyseur est détruit par addition d'un composé désactivateur, choisi parmi: a) les composés organiques A porteurs d'au moins un groupement hydroxyle, ou b) les composés résultant de la réaction de groupement isocyanate sur le composé organique A, le composé organique A étant éventuellement porteur de groupements ou atomes inertes vis-à-vis des groupements isocyanates et étant choisi parmi les énols, les alcools, les phénols, les oximes, ou c) les composés organosiliciques porteurs de groupement(s) hydroxysilylé(s).

2. Procédé selon la revendication 1, dans lequel le composé désactivateur est choisi parmi les composés organiques A ou les composés résultant de la réaction de groupement isocyanate sur le composé A, le composé A étant un énol, un alcool primaire, secondaire ou tertiaire, un polyol primaire, secondaire ou tertiaire, un phénol ou un polyphénol, une cétoxime, un silanol, un silanediol, un siloxane ou un polysiloxane à groupement(s) hydroxysilylé(s).

3. Procédé selon l'une des revendications 1 ou 2, dans lequel le composé désactivateur est choisi parmi les monoalcools primaires ou secondaires ayant de 1 à 8 atomes de carbone, ou parmi les uréthannes issus d'un composé porteur d'au moins un groupement isocyanate et d'un monoalcool primaire ou secondaire ayant de 1 à 8 atomes de carbone.

4. Procédé selon l'une des revendications 1 à 3, dans lequel le composé désactivateur est un monoalcool primaire ou secondaire ayant de 3 à 6 atomes de carbone.

5. Procédé selon l'une des revendications précédentes, dans lequel le composé aminosilylé utilisé à titre de catalyseur de cyclotrimérisation catalytique des polyisocyanates est un composé ayant pour formule:

$$R_{(4-n)}\text{———}Si-[NR'R'']_n \qquad (I)$$

dans laquelle les divers symboles repésentent respectivement:

– R: un radical monovalent de nature hydrocarbonée, aliphatique, cycloaliphatique, saturé ou insaturé, aryle, aralkyle ou alkylaryle, éventuellement substitué par des atomes d'halogène ou des groupements CN, deux radicaux R pouvant constituer ensemble un radical hydrocarboné divalent,

– R': un radical monovalent choisi parmi les radicaux R, $SiR_3$, ou les radicaux amide de formule:

$$-CO-\underset{\displaystyle R}{\underset{|}{N}}-R'''$$

R''' représentant R ou $SiR_3$, R ayant la signification préalablement donnée, le radical R' pouvant éventuellement, lorsqu'il ne représente pas un groupement amide ou un groupement $SiR_3$, constituer avec le radical R'' un radical hydrocarboné divalent,

– R'': un radical monovalent ayant la même signification que le radical R, ou un atome d'hydrogène lorsque R' n'est pas un radical amide,

– n: un nombre entier égal à 1 ou 2, lorsque n est égal à 2, R' est un radical R.

6. Procédé selon la revendication 5, dans lequel le composé à groupement aminosilylé a pour formule (I), dans laquelle les divers symboles représentent respectivement:

– R: un radical alkyle, alkényle ou halogénoalkyle ou halogénoalkényle ayant de 1 à 5 atomes de carbone et comportant de 1 à 6 atomes de chlore et/ou de fluor, des radicaux cycloalkyles, cycloalkényles et halogénocycloalkyles halogénocycloalkényles ayant de 3 à 8 atomes de carbone et contenant de 1 à 4 atomes de chlore et/ou de fluor; des radicaux aryles, alkylaryles et halogénoaryles ayant de 6 à 8 atomes de carbone et contenant de 1 à 4 atomes de chlore et/ou de fluor; des radicaux cyanoalkyles ayant de 3 à 4 atomes de carbone,

– deux symboles R portés par un même atome de silicium constituent entre eux un radical divalent ayant de 1 à 4 atomes de carbone,

– R': un radical monovalent choisi par les radicaux R, $SiR_3$, et $CO(NR)-R'''$, R''' représentant R ou $SiR_3$, R ayant la signification plus précise qui vient juste d'être donnée ci-dessus, R' pouvant constituer avec R'' un radical alkylène ayant de 4 à 6 atomes de carbone,

– R'': un radical alkyle ou alkényle ayant de 1 à 4 atomes de carbone, un radical cycloalkyle ou cycloalkényle ayant de 4 à 6 atomes de carbone nucléaires, un radical phényle ou tolyle ou xylyle, ou un atome d'hydrogène, lorsque R' n'est pas un groupement amide.

7. Procédé selon l'une des revendications 5 ou 6, dans lequel le composé à groupement aminosilylé est un aminosilane.

8. Procédé selon l'une des revendications 5 ou 6, dans lequel le composé à groupement aminosilylé est un diaminosilane.

9. Procédé selon l'une des revendications 5 ou 6, dans lequel le composé à groupement aminosilylé est une silylurée.

10. Procédé selon l'une des revendications 5 ou 6, dans lequel le composé à groupement aminosilylé est un silazane.

**Patentansprüche** für die Vertragsstaten BE, CH, DE, FR, GB, IT, LI, LU, NL, SE

1. Verfahren zur Herstellung von Polyisocyanatpolyisocyanuraten durch katalytische Cyclotrimerisation von Polyisocyanaten unter Verwendung von Verbindungen mit aminosilylhaltigen Gruppen als Katalysatoren, dadurch gekennzeichnet, dass, sobald der gewünschte Gehalt an Isocyanuratgruppen erreicht ist, der Katalysator durch Zusetzen einer Desaktivatorverbindung ausgewählt unter: a) den organischen Verbindungen A, die mindestens eine Hydroxylgruppe tragen, b) den Verbindungen, die bei der Reaktion einer Isocyanatgruppe mit der organischen Verbindung A entstehen, wobei die organische Verbindung A gegebenenfalls gegenüber Isocyanatgruppen inerte Gruppen oder Atome trägt, und ausgewählt ist unter den Enolen, den Alkoholen, den Phenolen, den Oximen, oder c) den Organosiliziumverbindungen, die eine hydroxysilylhaltige Gruppe oder hydroxsilylhaltige Gruppen tragen, zerstört wird.

2. Verfahren nach Anspruch 1, worin die Desaktivatorverbindung ausgewählt ist unter den organischen Verbindungen A oder den Verbindungen, die bei der Reaktion einer Isocyanatgruppe mit der Verbindung A entstehen, wobei die Verbindung A ein Enol, ein ein-, zwei- oder dreiwertiger Alkohol, ein primäres, sekundäres oder tertiäres Polyol, ein Phenol oder ein Polyphenol, ein Ketoxim, ein Silanol, ein Silandiol, ein Siloxan oder ein Polysiloxan mit einer hydroxysilylhaltigen Gruppe oder hydroxysilylhaltigen Gruppen ist.

3. Verfahren nach einem der Ansprüche 1 oder 2, worin die Desaktivatorverbindung ausgewählt ist unter den ein- oder zweiwertigen Monoalkoholen mit 1 bis 8 Kohlenstoffatomen oder unter den Urethanen, die von einer Verbindung abgeleitet sind, die mindestens eine Isocyanatgruppe und einen ein- oder zweiwertigen Monoalkohol mit 1 bis 8 Kohlenstoffatomen trägt.

4. Verfahren nach einem der Ansprüche 1 bis 3, wobei die Desaktivatorverbindung ein ein- oder zweiwertiger Monoalkohol mit 3 bis 6 Kohlenstoffatomen ist.

5. Verfahren nach einem der vorhergehenden Ansprüche, wobei die als Katalysator für die katalytische Cyclotrimerisation von Polyisocyanaten verwendete aminosilylhaltige Verbindung eine Verbindung der Formel ist

$$R_{(4-n)}\text{——}Si\text{-}[NR'R'']_n \qquad (I)$$

worin die verschiedenen Symbole sind

R ein einwertiger gesättigter oder ungesättigter aliphatischer oder cycloaliphatischer Kohlenwasserstoffrest, Aryl, Aralkyl oder Alkylaryl, gegebenenfalls substituiert durch Halogenatome oder CN-Gruppen, wobei zwei Reste R zusammen einen zweiwertigen Kohlenwasserstoffrest bilden können,

R' ein einwertiger Rest ausgewählt unter den Resten R, $SiR_3$ oder den Amidresten der Formel

$$-CO-\underset{\substack{|\\R}}{N}-R'''$$

worin R''' R oder $SiR_3$ ist, R die oben angegebene Bedeutung hat, der Rest R' gegebenenfalls, wenn er nicht eine Amidgruppe oder eine $SiR_3$-Gruppe ist, mit dem Rest R'' einen zweiwertigen Kohlenwasserstoffrest bilden kann,

R'' ein einwertiger Rest mit der gleichen Bedeutung wie der Rest R, oder ein Wasserstoffatom, wenn R' nicht ein Amidrest ist,

n eine ganze Zahl = 1 oder 2. Wenn n = 2, ist R' ein Rest R.

6. Verfahren nach Anspruch 5, wobei die Verbindung mit einer aminosilylhaltigen Gruppe der Formel (I) entspricht, worin die verschiedenen Symbole sind:

R ein Alkyl-, Alkenyl- oder Halogenalkyl- oder Halogenalkenylrest mit 1 bis 5 Kohlenstoffatomen und 1 bis 6 Chlor- und/oder Fluoratomen, Cycloalkyl-, Cycloalkenyl- und Halogencycloalkyl-, Halogencycloalkenylreste mit 3 bis 8 Kohlenstoffatomen und 1 bis 4 Chlor- und/oder Fluoratomen; Aryl-, Alkylaryl- und Halogenarylreste mit 6 bis 8 Kohlenstoffatomen und 1 bis 4 Chlor- und/oder Fluoratomen; Cyanoalkylreste mit 3 bis 4 Kohlenstoffatomen,

zwei von einem gleichen Siliziumatom getragene Symbole R bilden miteinander einen zweiwertigen Rest mit 1 bis 4 Kohlenstoffatomen,

R' ein einwertiger Rest ausgewählt unter den Resten R, $SiR_3$, und $CO(NR)-R'''$, wobei R''' R oder $SiR_3$ ist, R die präzisere Bedeutung hat, die gerade oben angegeben ist; R' mit R'' einen Alkylenrest mit 4 bis 6 Kohlenstoffatomen bilden kann,

R'' ein Alkyl- oder Alkenylrest mit 1 bis 4 Kohlenstoffatomen, ein Cycloalkyl- oder Cycloalkenylrest mit 4 bis 6 Kernkohlenstoffatomen ein Phenyl-, Tolyl- oder Xylylrest, oder ein Wasserstoffatom, wenn R' nicht eine Amidgruppe ist.

7. Verfahren nach einem der Ansprüche 5 oder 6, worin die Verbindung mit einer aminosilylhaltigen Gruppe ein Aminosilan ist.

8. Verfahren nach einem der Ansprüche 5 oder 6, wobei die Verbindung mit einer aminosilylhaltigen Gruppe ein Diaminosilan ist.

9. Verfahren nach einem der Ansprüche 5 und 6, wobei die Verbindung mit einer aminosilylhaltigen Gruppe ein Silylcarbamid ist.

10. Verfahren nach einem der Ansprüche 5 oder 6, wobei die Verbindung mit einer aminosilylhaltigen Gruppe ein Silazan ist.

11. Polyisocyanatpolyisocyanurate erhalten nach den Verfahren, wie sie in einem der Ansprüche 1 bis 10 angegeben sind.

**Patentansprüche** für den Vertragsstaat: AT

1. Verfahren zur Herstellung von Polyisocyanatpolyisocyanuraten durch katalytische Cyclotrimerisation von Polyisocyanaten unter Verwendung von Verbindungen mit aminosilylhaltigen Gruppen als Katalysatoren, dadurch gekennzeichnet, dass, sobald der gewünschte Gehalt an Isocyanuratgruppen erreicht ist, der Katalysator durch Zusetzen einer Desaktivatorverbindung ausgewählt unter: a) den organischen Verbindungen A, die mindestens eine Hydroxylgruppe tragen, b) den Verbindungen, die bei der Reaktion einer Isocyanatgruppe mit der organischen Verbindung A entstehen, wobei die organische Verbindung A gegebenenfalls gegenüber Isocyanatgruppen inerte Gruppen oder Atome trägt, und ausgewählt ist unter den Enolen, den Alkoholen, den Phenolen, den Oximen, oder c) den Organosiliziumverbindungen, die eine hydroxysilylhaltige Gruppe oder hydroxysilylhaltige Gruppen tragen, zerstört wird.

2. Verfahren nach Anspruch 1, worin die Desaktivatorverbindung ausgewählt ist unter den organischen Verbindungen A oder den Verbindungen, die bei der Reaktion einer Isocyanatgruppe mit der Verbindung A entstehen, wobei die Verbindung A ein Enol, ein ein-, zwei- oder dreiwertiger Alkohol, ein primäres, sekundäres oder tertiäres Polyol, ein Phenol oder ein Polyphenol, ein Ketoxim, ein Silanol, ein Silandiol, ein Siloxan oder ein Polysiloxan mit einer hydroxysilylhaltigen Gruppe oder hydroxysilylhaltigen Gruppen ist.

3. Verfahren nach einem der Ansprüche 1 oder 2, worin die Desaktivatorverbindung ausgewählt ist unter den ein- oder zweiwertigen Monoalkoholen mit 1 bis 8 Kohlenstoffatomen oder unter den Urethanen, die von einer Verbindung abgeleitet sind, die mindestens eine Isocyanatgruppe und einen ein- oder zweiwertigen Monoalkohol mit 1 bis 8 Kohlenstoffatomen trägt.

4. Verfahren nach einem der Ansprüche 1 bis 3, wobei die Desaktivatorverbindung ein ein- oder zweiwertiger Monoalkohol mit 3 bis 6 Kohlenstoffatomen ist.

5. Verfahren nach einem der vorhergehenden Ansprüche, wobei die als Katalysator für die katalytische Cyclotrimerisation von Polyisocyanaten verwendete aminosilylhaltige Verbindung eine Verbindung der Formel ist

$$R_{(4-n)}-Si[NR'R'']_n \quad (I)$$

worin die verschiedenen Symbole sind

R ein einwertiger gesättigter oder ungesättigter aliphatischer oder cycloaliphatischer Kohlenwasserstoffrest, Aryl, Aralkyl oder Alkylaryl, gegebenenfalls substituiert durch Halogenatome oder CN-Gruppen, wobei zwei Reste R zusammen einen zweiwertigen Kohlenwasserstoffrest bilden können,

R' ein einwertiger Rest ausgewählt unter den Resten R, $SiR_3$ oder den Amidresten der Formel

$$-CO-\underset{R}{\underset{|}{N}}-R'''$$

worin R''' R oder $SiR_3$ ist, R die oben angegebene Bedeutung hat, der Rest R' gegebenenfalls, wenn er nicht eine Amidgruppe oder eine $SiR_3$-Gruppe ist, mit dem Rest R'' einen zweiwertigen Kohlenwasserstoffrest bilden kann,

R'' ein einwertiger Rest mit der gleichen Bedeutung wie der Rest R; oder ein Wasserstoffatom, wenn R' nicht ein Amidrest ist,

n eine ganze Zahl = 1 oder 2. Wenn n = 2, ist R' ein Rest R.

6. Verfahren nach Anspruch 5, wobei die Verbindung mit einer aminosilylhaltigen Gruppe der Formel (I) entspricht, worin die verschiedenen Symbole sind

R ein Alkyl-, Alkenyl- oder Halogenalkyl- oder Halogenalkenylrest mit 1 bis 5 Kohlenstoffatomen und 1 bis 6 Chlor- und/oder Fluoratomen, Cycloalkyl-, Cycloalkenyl- und Halogencycloalkyl-, Halogencycloalkenylreste mit 3 bis 8 Kohlenstoffatomen und 1 bis 4 Chlor- und/oder Fluoratomen; Aryl-, Alkylaryl- und Halogenarylreste mit 6 bis 8 Kohlenstoffatomen und 1 bis 4 Chlor- und/oder Fluoratomen; Cyanoalkylreste mit 3 bis 4 Kohlenstoffatomen,

zwei von einem gleichen Siliziumatom getragene Symbole R bilden miteinander einen zweiwertigen Rest mit 1 bis 4 Kohlenstoffatomen,

R' ein einwertiger Rest ausgewählt unter den Resten R, $SiR_3$, und CO(NR)−R''', wobei R''' R oder $SiR_3$ ist, R die präzisere Bedeutung hat, die gerade oben angegeben ist; R' mit R'' einen Alkylenrest mit 4 bis 6 Kohlenstoffatomen bilden kann,

R'' ein Alkyl- oder Alkenylrest mit 1 bis 4 Kohlenstoffatomen, ein Cycloalkyl- oder Cycloalkenylrest mit 4 bis 6 Kernkohlenstoffatomen, ein Phenyl-, Tolyl- oder Xylylrest, oder ein Wasserstoffatom, wenn R' nicht eine Amidgruppe ist.

7. Verfahren nach einem der Ansprüche 5 oder 6, worin die Verbindung mit einer aminosilylhaltigen Gruppe ein Aminosilan ist.

8. Verfahren nach einem der Ansprüche 5 und 6, wobei die Verbindung mit einer aminosilylhaltigen Gruppe ein Diaminosilan ist.

9. Verfahren nach einem der Ansprüche 5 und 6, wobei die Verbindung mit einer aminosilylhaltigen Gruppe ein Silylcarbamid ist.

10. Verfahren nach einem der Ansprüche 5 oder 6, wobei die Verbindung mit einer aminosilylhaltigen Gruppe ein Silazan ist.

**Claims** for the Contracting States BE, CH, DE, FR, GB, IT, LI, LU, NL, SE

1. Process for the preparation of polyisocyanato-polyisocyanurates by the catalytic cyclotrimerisation of polyisocyanates using compounds containing aminosilyl groups as catalysts, characterised in that, when the desired proportion of

isocyanurate groups is reached, the catalyst is destroyed by the addition of a deactivating compound chosen from amongst

(*a*) organic compounds A carrying at least one hydroxyl group, or

(*b*) compounds resulting from the reaction of an isocyanate group with the organic compounds A, the organic compound A optionally carrying groups or atoms which are inert towards the isocyanate groups and being chosen from amongst enols, alcohols, phenols, oximes, or

(*c*) organosilicon compounds carrying hydroxysilyl group(s).

2. Process according to Claim 1, in which the deactivating compound is chosen from amongst organic compounds A, or compounds resulting from the reaction of an isocyanate group with the compound A, the compound A being an enol, primary, secondary or tertiary alcohol, a primary, secondary or tertiary polyol, a phenol or a polyphenol, a ketoxime, a silanol, a silanediol, a siloxane or a polysiloxane containing hydroxysilyl group(s).

3. Process according to Claim 1 or 2, in which the deactivating compound is chosen from amongst primary or secondary monoalcohols having from 1 to 8 carbon atoms or from amongst urethanes derived from a compound carrying at least one isocyanate group and from a primary or secondary monoalcohol having from 1 to 8 carbon atoms.

4. Process according to one of Claims 1 to 3, in which the deactivating compound is a primary or secondary monoalcohol having from 3 to 6 carbon atoms.

5. Process according to one of the preceding claims, in which the aminosilyl compound used as the catalyst for the catalytic cyclotrimerisation of the polyisocyanates is a compound having the formula

$$R_{(4-n)}-Si\left[NR'R''\right]_n \qquad (I)$$

in which the various symbols respectively represent the following

R a monovalent radical of hydrocarbon type which is aliphatic or cycloaliphatic, saturated or unsaturated and an aryl, aralkyl or alkylaryl radical, and which is optionally substituted by halogen atoms or CN groups, it being possible for two radicals R together to form a divalent hydrocarbon radical,

R′ a monovalent radical chosen from amongst the radicals R and $SiR_3$ or the amide radicals of the formula

$$-CO-\underset{\substack{|\\ R}}{N}-R'''$$

R‴ representing R or $SiR_3$, R having the meaning given above and it optionally being possible for the radical R′, if it does not represent an amide group or a group $SiR_3$, to form a divalent hydrocarbon radical with the radical R″,

R″ a monovalent radical having the same meaning as the radical R, or a hydrogen atom if R′ is not an amide radical, and

n an integer equal to 1 or 2; if n is equal to 2, R′ is a radical R.

6. Process according to Claim 5, in which the compound containing an aminosilyl group has the Formula (I) in which the various symbols respectively represent the following

R an alkyl, alkenyl, halogenoalkyl or halogenoalkenyl radical having from 1 to 5 carbon atoms and optionally containing from 1 to 6 chlorine and/or fluorine atoms, a cycloalkyl, cycloalkenyl, halogenocycloalkyl or halogenocycloalkenyl radical having from 3 to 8 carbon atoms and optionally containing from 1 to 4 chlorine and/or fluorine atoms, an aryl, alkylaryl or halogenoaryl radical having from 6 to 8 carbon atoms and optionally containing from 1 to 4 chlorine and/or fluorine atoms, or a cyanoalkyl radical having from 3 to 4 carbon atoms,

two symbols R carried by the same silicon atom together form a divalent radical having from 1 to 4 carbon atoms,

R′ a monovalent radical chosen from amongst the radicals R, $SiR_3$ and $-CO(NR)-R'''$, R‴ representing R or $SiR_3$, R having the more precise meaning which has just been given above, and it being possible for R′ to form with R″ an alkylene radical having from 4 to 6 carbon atoms, and

R″ an alkyl or alkenyl radical having from 1 to 4 carbon atoms, a cycloalkyl or cycloalkenyl radical having from 4 to 6 nuclear carbon atoms, a phenyl, tolyl or xylyl radical, or a hydrogen atom if R′ if not an amide group.

7. Process according to Claim 5 or 6, in which the compound containing an aminosilyl group is an aminosilane.

8. Process according to Claim 5 or 6, in which the compound containing an aminosilyl group is a diaminosilane.

9. Process according to Claim 5 or 6, in which the compound containing an aminosilyl group is a silylurea.

10. Process according to Claim 5 or 6, in which the compound containing an aminosilyl group is a silazane.

11. Polyisocyanato-polyisocyanurates obtained by the processes such as defined in one of Claims 1 to 10.

**Claims** for the Contracting state: AT

1. Process for the preparation of polyisocyanato-polyisocyanurates by the catalytic cyclotrimerisation of polyisocyanates using compounds containing aminosilyl groups as catalysts, characterised in that, when the desired proportion of isocyanurate groups is reached, the catalyst is destroyed by the addition of a deactivating compound chosen from amongst

(*a*) organic compounds A carrying at least one hydroxyl group, or

(*b*) compounds resulting from the reaction of an isocyanate group with the organic compounds

A, the organic compound A optionally carrying groups or atoms which are inert towards the isocyanate groups and being chosen from amongst enols, alcohols, phenols, oximes, or

(*c*) organosilicon compounds carrying hydroxysilyl group(s).

2. Process according to Claim 1, in which the deactivating compound is chosen from amongst organic compounds A or compounds resulting from the reaction of an isocyanate group with the compound A, the compound A being an enol, primary, secondary or tertiary alcohol, a primary, secondary or tertiary polyol, a phenol or a polyphenol, a ketoxime, a silanol, a silanediol, a siloxane or a polysiloxane containing hydroxysilyl group(s).

3. Process according to Claim 1 or 2, in which the deactivating compound is chosen from amongst primary or secondary monoalcohols having from 1 to 8 carbon atoms or from amongst urethanes derived from a compound carrying at least one isocyanate group and from a primary or secondary monoalcohol having from 1 to 8 carbon atoms.

4. Process according to Claims 1 to 3, in which the deactivating compound is a primary or secondary monoalcohol having from 3 to 6 carbon atoms.

5. Process according to one of the preceding claims, in which the aminosilyl compound used as the catalyst for the catalytic cyclotrimerisation of the polyisocyanates is a compound having the formula

$$R_{(4-n)}-Si[NR'R'']_n \quad (I)$$

in which the various symbols respectively represent the following

R a monovalent radical of hydrocarbon type which is aliphatic or cycloaliphatic, saturated or unsaturated and an aryl, aralkyl or alkylaryl radical, and which is optionally substituted by halogen atoms or CN groups, it being possible for two radicals R together to form a divalent hydrocarbon radical,

R' a monovalent radical chosen from amongst the radical R and $SiR_3$ or the amide radicals of the formula

$$-CO-\underset{\displaystyle R}{\underset{|}{N}}-R'''$$

R''' representing R or $SiR_3$, R having the meaning given above and it optionally being possible for the radical R', if it does not represent an amide group or a group $SiR_3$, to form a divalent hydrocarbon radical with the radical R'',

R'' a monovalent radical having the same meaning as the radical R, or a hydrogen atom if R' is not an amide radical, and

n an integer equal to 1 or 2; if n is equal to 2, R' is a radical R.

6. Process according to Claim 5, in which the compound containing an aminosilyl group has the Formula (I) in which the various symbols respectively represent the following

R an alkyl, alkenyl, halogenoalkyl or halogenoalkenyl radical having from 1 to 5 carbon atoms and optinally containing from 1 to 6 chlorine and/or fluorine atoms, a cycloalkyl, cycloalkenyl, halogenocycloalkyl or halogenocycloalkenyl radical having from 3 to 8 carbon atoms and optionally containing from 1 to 4 chlorine and/or fluorine atoms, an aryl, alkylaryl or halogenoaryl radical having from 6 to 8 carbon atoms and optionally containing from 1 to 4 chlorine and/or fluorine atoms, or a cyanoalkyl radical having from 3 to 4 carbon atoms,

two symbols R carried by the same silicon atom together form a divalent radical having from 1 to 4 carbon atoms,

R' a monovalent radical chosen from amongst the radicals R, $SiR_3$ and $-CO(NR)-R'''$, R''' representing R or $SiR_3$, R having the more precise meaning which has just been given above, and it being possible for R' to form with R'' an alkylene radical having from 4 to 6 carbon atoms, and

R'' an alkyl or alkenyl radical having from 1 to 4 carbon atoms, a cycloalkyl or cycloalkenyl radical having from 4 to 6 nuclear carbon atoms, a phenyl, tolyl or xylyl radical, or a hydrogen atom if R' is not an amide group.

7. Process according to Claim 5 or 6, in which the compound containing an aminosilyl group is an aminosilane.

8. Process according to Claim 5 or 6, in which the compound containing an aminosilyl group is a diaminosilane.

9. Process according to Claim 5 or 6, in which the compound containing an aminosilyl group is a silylurea.

10. Process according to Claim 5 or 6, in which the compound containing an aminosilyl group is a silazane.